(19) Europäisches Patentamt
European Patent Office
Office européen des brevets

(11) Veröffentlichungsnummer : **0 008 734**
**B1**

(12) # EUROPÄISCHE PATENTSCHRIFT

(45) Veröffentlichungstag der Patentschrift : **29.07.81**

(21) Anmeldenummer : **79103047.1**

(22) Anmeldetag : **20.08.79**

(51) Int. Cl.$^3$ : **C 07 C 43/29**, C 07 C 41/24// C07C121/75, C07C120/00, C07C41/22, C07C47/575, C07C45/27

(54) **Verfahren zur Herstellung von 4-Fluoro-3-phenoxy-toluol.**

(30) Priorität : **28.08.78 DE 2837525**

(43) Veröffentlichungstag der Anmeldung : **19.03.80 (Patentblatt 80/06)**

(45) Bekanntmachung des Hinweises auf die Patenterteilung : **29.07.81 Patentblatt 81/30**

(84) Benannte Vertragsstaaten : **BE CH DE FR GB IT NL**

(56) Entgegenhaltungen :
**DE - A - 2 619 489**
**FR - A - 1 549 827**
**FR - A - 2 085 713**
**US - A - 3 391 195**
**US - A - 3 454 392**

(73) Patentinhaber : **BAYER AG**
**Zentralbereich Patente, Marken und Lizenzen**
**D-5090 Leverkusen 1, Bayerwerk (DE)**

(72) Erfinder : **Cölln, Reimer, Dr.**
**In den Birken 67**
**D-5600 Wuppertal 1 (DE)**
Erfinder : **Diehr, Hans-Joachim, Dr.**
**Höhe 35**
**D-5600 Wuppertal 1 (DE)**
Erfinder : **Priesnitz, Uwe, Dr.**
**Heinrich-Heine-Strasse 42**
**D-4750 Unnamassen (DE)**

**EP 0 008 734 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zur Herstellung von 4-Fluoro-3-phenoxy-toluol

Die Erfindung betrifft ein neues Verfahren zur Herstellung von 4-Fluoro-3-phenoxy-toluol.

Es sind Reaktionen von Halogeno-fluoro-benzolderivaten mit Alkoholaten bzw. Phenolaten, bei denen Fluor eher als andere Halogene substituiert wird, bekannt geworden. So entsteht z.B. aus 4-Fluorochlorbenzol und Kaliumphenolat als Hauptprodukt 4-Chloro-diphenyläther (vergleiche DE-OS 2 619 489). Die Reaktion von Chloropentafluoro-benzol mit Natriumpentafluorophenolat führt zu einem Isomerengemisch aus 4-Chloro-nonafluoro-diphenyläther und 2-Chloro-nonafluoro-diphenyläther (vgl. US-PS 3 637 866).

Bromo-2,3,4,6-tetrafluoro-benzol reagiert mit Natrium-methylat zu einem Isomerengemisch von Bromo-trifluoro-methoxy-benzolen (vgl. J. Chem. Soc. Perkin Trans. II *1978*, S. 137-141).

Bekannt sind auch mehrere Verfahren zur Herstellung von 3-Phenoxy-toluol durch Umsetzung von Brombenzol mit m-Cresolat ; dabei stellt sich jedoch nicht das Problem der selektiven Substitution eines von zwei verschiedenen Halogensubstituenten (vergleiche GB-PS 1 052 390 und DE-OS 2 619 489).

Ferner ist bekannt, daß es zweckmäßig sein kann, die Herstellung von Diphenyläthern aus Halogenobenzolen und Phenolaten in Gegenwart von Kaliumsalzen durchzuführen (vergleiche DE-OS 2 242 519).

Es wurde nun gefunden, daß man 4-Fluoro-3-phenoxy-toluol der Formel I

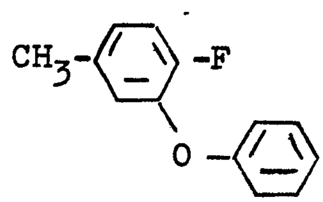

(I)

in guten Ausbeuten und hoher Reinheit erhält, wenn man 3-Bromo-4-fluoro-toluol der Formel II

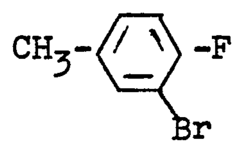

(II)

mit Kaliumphenolat oder mit Natriumphenolat in Gegenwart eines Kaliumsalzes jeweils in Gegenwart von Kupfer oder einer katalytisch wirksamen Kupferverbindung unter Verwendung eines Verdünnungsmittels aus der Reihe der Carbonsäureamide bei Temperaturen zwischen 120 und 180 °C umsetzt.

Vorteile des neuen Verfahrens sind seine relativ einfache Durchführbarkeit sowie die gute Ausbeute und hohe Reinheit des Produktes.

Die erfindungsgemäße Reaktion kann durch folgendes Formelschema skizziert werden :

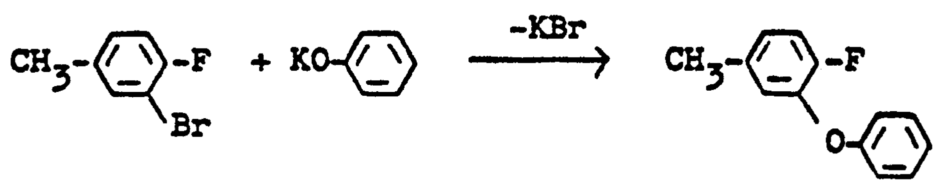


Das als Ausgangsverbindung zu verwendende 3-Bromo-4-fluoro-toluol ist bereits bekannt (vergleiche Canad. Journ. Chem. *38* (1960), 2 441-2 449).

Das erfindungsgemäße Verfahren wird im allgemeinen unter Verwendung von Verdünnungsmitteln aus der Reihe der Carbondäureamide wie z. B. Dimethylformamid, Dimethylacetamid und N-Methylpyrrolidon, durchgeführt. Besonders bewährt hat sich Dimethylacetamid.

Als Katalysatoren werden Kupfer oder Kupferverbindungen verschiedener Oxidationsstufen verwendet. Beispielsweise seien Kupfer, Kupfer (I)- und -(II) oxid, Kupfer (I) chlorid und Kupfer (II) chlorid, Kupfer (I) bromid und Kupfer (II) bromid sowie Kupfer (I) iodid genannt.

Die Reaktionstemperatur liegt beim erfindungsgemäßen Verfahren im allgemeinen zwischen 120 und 180 °C, vorzugsweise zwischen 130 und 170 °C. Die Umsetzung wird im allgemeinen bei Normaldruck durchgeführt.

Auf 1 Mol 3-Bromo-4-fluoro-toluol setzt man beim Arbeiten mit Kaliumphenolat im allgemeinen zwischen 1,5 und 8 Mol, vorzugsweise zwischen 2 und 4 Mol Kaliumphenolat ein. In einer besonderen Ausführungsform des Verfahrens wird das Kaliumphenolat aus äquimolaren Mengen Kaliumhydroxid und Phenol in situ vor der Umsetzung mit 3-Bromo-4-fluorotoluol hergestellt. Die Umsetzung wird im

allgemeinen in einem der angegebenen Verdünnungsmittel in Gegenwart eines der angegebenen Katalysatoren durchgeführt; man setzt hierzu zwischen 0,001 und 0,1 Mol, vorzugsweise zwischen 0,005 und 0,05 Mol Katalysator je Mol 3-Bromo-4-fluorotoluol ein. Das Reaktionsgemisch wird mehrere Stunden, im allgemeinen zwischen drei und fünf Stunden lang, bei der erforderlichen Temperatur gerührt.

Zur Aufarbeitung wird die auf etwa 100 °C abgekühlte Mischung mit Wasser versetzt und mehrmals mit einem mit Wasser nicht mischbaren Lösungsmittel geschüttelt. Die vereinigten organischen Extrakte werden mit verdünnter Natronlauge und Wasser gewaschen und nach Abziehen des Lösungsmittels im Vakuum wird das zurückbleibende Rohprodukt durch Vakuumdestillation gereinigt. Als Reinheitskriterien dienen Gaschromatogramm, Siedepunkt und Brechnungsindex.

Auf 1 Mol 3-Bromo-4-fluoro-toluol setzt man beim Arbeiten mit Natriumphenolat in Gegenwart eines Kaliumsalzes im allgemeinen zwischen 1 und 3, vorzugsweise zwischen 1,05 und 1,5 Mol Natriumphenolat und zwischen 0,1 und 2, vorzugsweise zwischen 0,4 und 1,5 Mol Kaliumsalz, vorzugsweise Kaliumchlorid, ein. Die Umsetzung wird in einem der oben angegebenen Lösungsmittel bei einer Temperatur zwischen 120 und 150 °C gestartet und anschließend bei 130 bis 170 °C weitergeführt. Der Katalysator wird in einer Menge zwischen 0,001 und 0,1 Mol, vorzugsweise zwischen 0,005 und 0,05 Mol je Mol 3-Bromo-4-fluoro-benzol eingesetzt. Das Reaktionsgemisch wird im allgemeinen zwischen 12 und 48 Stunden, vorzugsweise zwischen 20 und 30 Stunden, bei der erforderlichen Temperatur gerührt.

Zur Aufarbeitung wird die abgekühlte Mischung mit einem nicht mit Wasser mischbaren Lösungsmittel und Kieselgur verrührt und abgesaugt. Man wäscht das Filtrat mit verdünnter Natronlauge und Wasser, zieht das Lösungsmittel im Vakuum ab und destilliert das zurückbleibende Rohprodukt im Hochvakuum. Als Reinheitskriterien dienen Gaschromatogramm, Siedepunkt und Brechungsindex.

Das nach dem erfindungsgemäßen Verfahren hergestellte 4-Fluoro-3-phenoxy-toluol kann als Zwischenprodukt zur Herstellung insektizid und akarizid wirksamer 4-Fluoro-3-phenoxy-benzyloxy-carbonylverbindungen der Formel

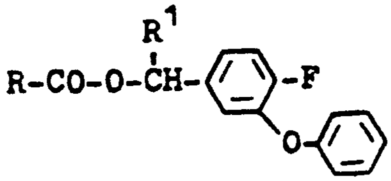 (III)

in welcher

R für den Rest 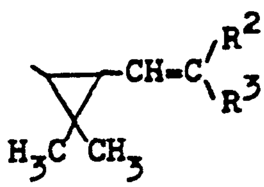

steht, wobei $R^2$ und $R^3$ gleich oder verschieden sind und für Chlor, Brom oder Methyl stehen, oder in welcher R für den Rest

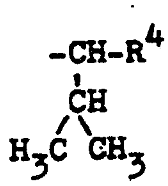

steht, wobei $R^4$ für gegebenenfalls durch Halogen, Alkyl, Alkoxy, Alkylthio, Nitro oder Methylendioxy substituiertes Phenyl steht und $R^1$ für Wasserstoff, Cyano oder Athinyl steht, verwendet werden (vergleiche DE-OS 2 709 264).

Verbindungen der Formel (III) erhält man in allgemeinen durch Umsetzung von Carbonsäurechloriden der Formel

R–CO–Cl (IV)

in welcher R die oben angegebene Bedeutung hat, mit 4-Fluoro-3-phenoxy-benzylalkoholen der Formel

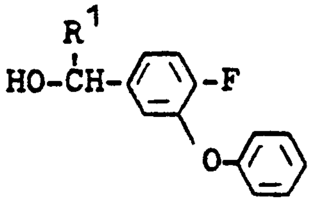

(V)

in welcher R[1] die oben angegebene Bedeutung hat, gegebenenfalls in Gegenwart eines Säureakzeptors und gegebenenfalls unter Verwendung eines Verdünnungsmittels.

Beispielsweise erhält man 2,2-Dimethyl-3-(2,2-dichlorvinyl)-cyclopropancarbonsäure-∝-cyano-4-fluoro-3-phenoxy-benzylester durch Umsetzung von 2,2-Dimethyl-3-(2,2-dichlorvinyl)-cyclopropancarbonsäurechlorid mit ∝-Cyano-4-fluoro-3-phenoxy-benzylalkohol in Gegenwart äquimolarer Mengen Pyridin als Säureakzeptor und unter Verwendung von Toluol als Verdünnungsmittel bei Temperaturen zwischen 0 und 50 °C, vorzugsweise bei 20 bis 30 °C gemäß folgenden Formelschema :

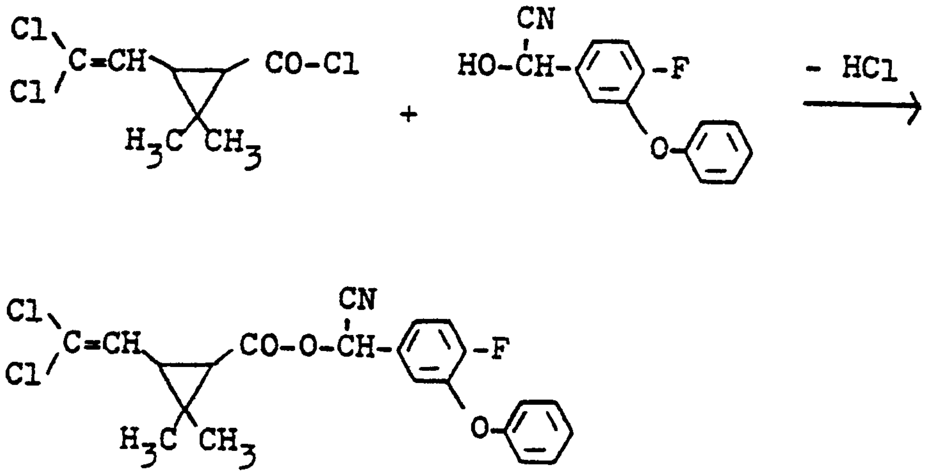


4-Fluoro-3-phenoxy-benzylalkohole der Formel (V) können aus dem erfindungsgemäß herzustellenden 4-Fluoro-3-phenoxy-toluol nach verschiedenen literaturbekannten Methoden über mehrere Stufen hergestellt werden.

Beispielsweise erhält man aus 4-Fluoro-3-phenoxy-toluol durch Umsetzung mit N-Bromsuccinimid in Gegenwart eines Radikalstarters wie z.B. Azodiisobuttersäurenitril, gegebenenfalls unter Verwendung eines Verdünnungsmittels wie z.B. Tetrachlorkohlenstoff, bei Temperaturen zwischen 50 und 100 °C 4-Fluoro-3-phenoxy-benzylbromid. Daraus kann in einer Sommelet-Reaktion, d.h. durch Umsetzung mit Hexamethylentetramin in Gegenwart eines Verdünnungsmittels wie z.B. Methylenchlorid bei Temperaturen zwischen 0 und 50 °C, anschließend mit wässriger Essigsäure und im Amschlub daran mit Salzsäure bei Temperaturen zwischen 80 und 120 °C 4-Fluoro-3-phenoxy-benzaldehyd hergestellt werden. Dessen Umsetzung mit Natriumcyanid in wässriger Essigsäure bei 20 °C liefert ∝-Cyano-4-fluoro-3-phenoxy-benzylalkohol. Die hier beschriebene Reaktionsfolge ist in folgendem Formelschema skizziert :

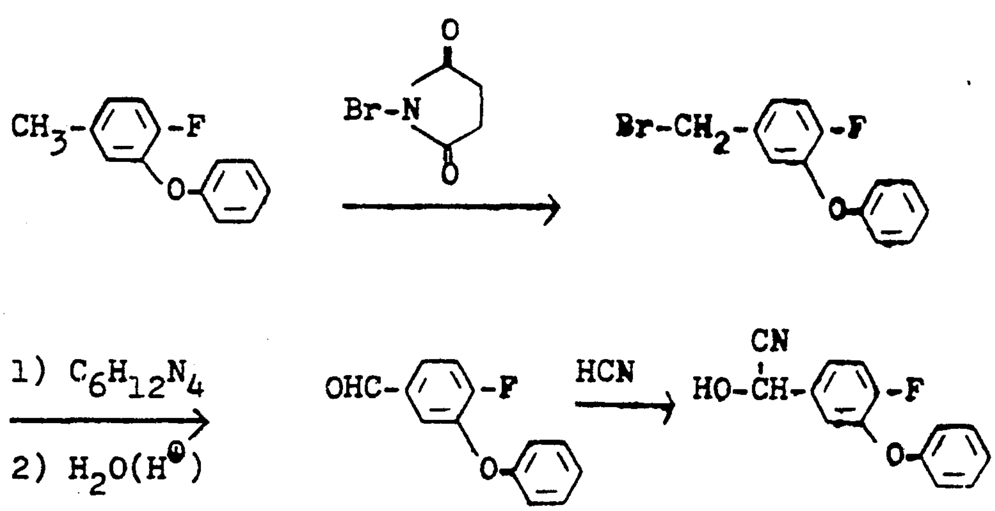

4-Fluoro-3-phenoxybenzylalkohol bzw. α-Äthinyl-4-fluoro-3-phenoxy-benzylalkohol erhält man aus 4-Fluoro-3-phenoxybenzaldehyd beispielsweise durch Umsetzung mit Lithiumaluminiumhydrid bzw. Athinylmagnesiumbromid nach bekannten Verfahren.

Die Verwendung der ausgehend von 4-Fluoro-3-phenoxy-toluol herzustellenden 4-Fluoro-3-phenoxybenzyloxy-carbonylverbindungen der Formel (III) als Insektizide und Akarizide ist Gegenstand einer älteren (nicht vorveröffentlichen Anmeldung der Anmelderin (vgl. DE-OS 2 709 264).

Beispiel 1

In einem 1l-Dreihalskolben werden 128 g N,N-Dimethyl-acetamid auf 130 bis 140 °C erhitzt. Unter Rühren trägt man bei dieser Temperatur 128 g Natriumphenolat (1,1 Mol) ein. Zur Lösung gibt man weiterhin 76 g Kaliumchlorid und 1 g Kupferoxyd. Zu der gerührten Mischung läßt man innerhalb von ca. 10 Minuten 189,1 g (1 Mol) 3-Bromo-4-fluoro-toluol bei 140 °C hinzufließen. Während 24 Stunden wird unter Rühren eine Temperatur von 150-155 °C aufrechterhalten. Nach dem Abkühlen verrührt man den Kolbeninhalt mit 800 ml Ligroin und 40 g Kieselgur. Es wird abgesaugt, der Filterrückstand mit Ligroin nachgewaschen und das Filtrat mit 400 ml 10 %iger Natronlauge geschüttelt. Nach dem Neutralwaschen mit Wasser wird das Lösungsmittel im Vakuum entfernt und der Rückstand im Vakuum destilliert; man erhält so 142,6 g farbloser Flüssigkeit vom $Kp._2$ 94-96 °C ($n_D^{22}$ : 1,5559). Die Ausbeute beträgt 70,5 % der Theorie an 4-Fluoro-3-phenoxy-toluol, bezogen auf eingesetztes Bromo-fluorotoluol.

Beispiel 2

In einem 1l-Rührkolben werden 141,2 g (1,5 Mol) Phenol, 1,5 Mol Kaliumhydroxid und 500 ml Xylol gemischt. Die Mischung wird durch Kochen am Wasserabscheider entwässert, bis ca. 32 ml Wasser abgeschieden sind. Man destilliert dann im Vakuum das Lösungsmittel ab und setzt 150 ml N,N-Dimethylacetamid und 0,5 g Kupferoxyd hinzu. Bei einer Innentemperatur von 140-145 °C läßt man unter Rühren im Laufe von ca. 5 Minuten 94,2 g (0,5 Mol) 3-Bromo-4-fluoro-toluol hineinfließen und rührt noch 4 Stunden bei dieser Temperatur nach. Man kühlt auf 100 °C ab und setzt 350 ml Wasser hinzu. Die Mischung wird bei Raumtemperatur dreimal mit je 200 ml Ligroin ausgeschüttelt und der gesammelte Extrakt mit verdünnter Natronlauge und Wasser gewaschen. Das Lösungsmittel wird im Vakuum abgezogen und der Rückstand destilliert. Man erhält so 81,9 g einer farblosen Flüssigkeit vom $Kp._2$ 94-96 °C ($n_D^{23}$ : 1,5555). Die Ausbeute beträgt 81 % der Theorie an 4-Fluoro-3-phenoxy-toluol, bezogen auf eingesetztes Bromofluorotoluol.

**Ansprüche**

1. Verfahren zur Herstellung von 4-Fluoro-3-phenoxy-toluol der Formel I

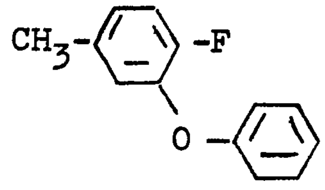

(I)

durch Umsetzung von 3-Bromo-4-fluorotoluol der Formel II

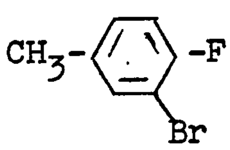

(II)

mit Kaliumphenolat oder mit Natriumphenolat in Gegenwart eines Kaliumsalzes jeweils in Gegenwart von Kupfer oder einer katalytisch wirksamen Kupferverbindung unter Verwendung eines Verdünnungsmittels aus der Reihe der Carbonsäureamide bei Temperaturen zwischen 120 und 180 °C.

2. Verfahren gemäß Anspruch 1, dadurch gekennzeichnet, daß als Lösungsmittel Dimethylformamid oder Dimethylacetamid verwendet wird.

**Claims**

1. A process for the preparation of 4-fluoro-3-phenoxy-toluene of the formula I

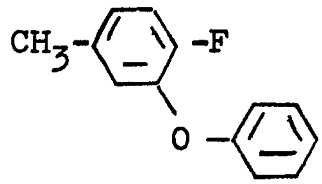

(I)

by reacting 3-bromo-4-fluoro-toluene of the formula II

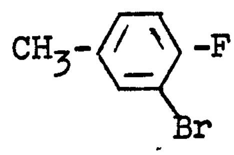

(II)

with potassium phenolate or with sodium phenolate in the presence of a potassium salt, in either case in the presence of copper or of a catalytically active copper compound, using a diluent from the group of carboxylic acid amides at temperatures between 120 and 180 °C.

2. A process according to claim 1, characterised in that dimethyl formamide or dimethyl acetamide is used as the solvent.

**Revendications**

1. Procédé de fabrication de 4-fluoro-3-phénoxy-toluène de formule I

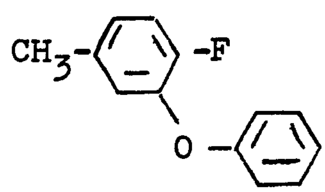

(I)

par réaction du 3-bromo-4-fluorotoluène de formule II

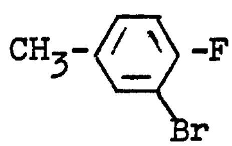

(II)

avec du phénolate de potassium ou avec du phénolate de sodium en présence d'un sel de potassium et chaque fois en présence de cuivre ou d'un composé de cuivre catalytiquement actif, avec utilisation d'un diluant de la série des carboxamides, à des températures entre 120 et 180 °C.

2. Procédé selon la revendication 1, caractérisé en ce qu'on utilise comme solvant de la diméthylformamide ou de la diméthylacétamide.